# FASCICULE DE BREVET EUROPEEN

(11) **EP 1 075 289 B1**
(45) Date de publication et mention de la délivrance du brevet: **05.12.2001**
(21) Numéro de dépôt: 99915803.3
(22) Date de dépôt: 20.04.1999
(51) Int. Cl.: A61L 31/00

(54) **IMPLANTS DE RENFORT POUR SUTURES TISSULAIRES**
VERSTÄRKUNGSIMPLANTAT FÜR GEWEBEÄHNLICHE NÄHFADEN
REINFORCING IMPLANTS FOR TISSUE SUTURES

(30) Priorité: 29.04.1998 FR 9805688
(43) Date de publication de la demande: 14.02.2001
(73) Titulaire: Thevenet, Fabrice, 69500 Bron (FR)
(72) Inventeur: Thevenet, Fabrice, 69500 Bron (FR)
(74) Mandataire: Benech, Frédéric
(86) Numéro de dépôt international: FR9900934
(87) Numéro de publication internationale: WO9955397

(56) Documents cités:
- EP-A- 0 213 563
- EP-A- 0 262 890
- US-A- 4 668 224

## Description

L'invention concerne un implant destiné à assurer le renfort mécanique, notamment au niveau de sutures tissulaires, manuelles ou mécaniques.

Lors d'interventions chirurgicales, en particulier dans le domaine de la chirurgie thoracique, il est habituel soit de sectionner et donc d'enlever une partie de certains organes, qu'il convient ensuite de refermer par des sutures, soit de réparer des structures existantes, et ce plus particulièrement dans le domaine cardio-vasculaire.

Dans un cas comme dans l'autre, il est procédé à des sutures manuelles ou mécaniques, (au moyen d'une pince à deux branches dont l'une est munie d'agrafes, l'autre servant d'enclume), destinées à circonscrire toutes fuites, qu'elles soient de nature sanguine ou aérique, notamment dans le cadre de la chirurgie pulmonaire. Cette étanchéité vis à vis du sang et/ou de l'air, pose un véritable problème au praticien, dans la mesure où les sutures effectuées fragilisent les tissus, qui ont tendance à se déchirer, ces tissus étant en outre déjà fragilisés par la pathologie. Cette fragilité entraîne des fuites d'air dans le cadre du poumon, et de manière plus générale de sang, nécessitant la mise en place et la maintien de drains, conduisant, en cas de fuites prolongées, à une augmentation de la durée d'hospitalisation du patient, et partant, à une augmentation significative des coûts correspondants.

Afin de pallier ces inconvénients majeurs, on a proposé de mettre en place des implants de renforcement réalisés en PTFE (polytétrafluoroéthylène) et, se présentant sous la forme d'une gaine ou d'un manchon cylindrique ou parallélépipèdique, destinés à venir se mettre en place par simple coulissement sur les deux branches d'une pince suturante de type en soi connu. L'actionnement d'une telle pince induit la pose d'agrafes de part et d'autre de la ligne de suture, venant ainsi fixer ledit manchon au niveau de l'organe en question. Sur certains types de pince, cet actionnement induit en outre le sectionnement de l'organe et du manchon, de sorte que ne subsiste au niveau de l'organe résiduel qu'une partie dudit manchon, faisant office de renfort de suture à type d'attelle.

Il existe également des attelles planes en PTFE, qui, découpées en bandes, renforcent les sutures manuelles réalisées au moyen d'un fil résorbable ou non.

Si certes, la mise en place d'un tel implant provoque le renfort mécanique de la suture, en revanche, outre son prix tout particulièrement élevé, un tel implant n'est pas bio-résorbable. Cet inconvénient est particulièrement ennuyeux en cas d'infection.

Il a également été proposé la mise en oeuvre, de la même façon, d'attelles planes ou cylindriques, réalisées à partir de péricarde bovin fixé au glutaraldéhyde. De même que dans le cas précédent, on se heurte avec un tel implant à un prix élevé. Par ailleurs et surtout, ces produits sont d'une innocuité virale incertaine, et surtout d'une innocuité vis à vis du prion tout à fait discutable et aléatoire.

En d'autres termes, la mise en oeuvre de telles attelles peut servir de vecteur à la transmission de virus ou de prions et s'avèrent donc de ce fait dangereuse. Par ailleurs, de telles attelles ne sont également pas bio-résorbables.

L'objet de l'invention est de proposer des gaines de renfort pour sutures tissulaires, qui présentent à la fois des propriétés de bio-résorption, d'élasticité et de malléabilité qui permettent une adaptation parfaite au support tissulaire et optimisent l'étanchéité gazeuse et/ou sanguine des sutures, et cela à un prix de revient raisonnable.

L'objet de l'invention est également de proposer une gaine de renfort qui présente une innocuité totale, après stérilisation, concernant la transmission des virus ou des prions.

Un autre objet de l'invention est de proposer une gaine de renfort pour sutures tissulaires susceptible de favoriser la cicatrisation des tissus ainsi protégés, dans le but de réduire les durées de drainage, d'hospitalisation, donc les coûts, et de ne pas constituer un facteur aggravant en cas d'infection. Cet implant résorbable permet d'obtenir une réduction de la réaction inflammatoire.

Enfin, l'invention vise également à proposer une gaine de renfort pour sutures tissulaires, susceptible de présenter une certaine étanchéité, notamment par rapport à l'air et aux liquides biologiques (sang, lymphe, sucs digestifs) au niveau des organes ainsi suturés.

Cette gaine de renfort de sutures tissulaires destiné à être mis en oeuvre pour renforcer mécaniquement les zones tissulaires suturées, se caractérise en ce qu'il est constitué d'une nappe textile réalisée à base d'acide polyuronique ou de ses sels.

Cette nappe textile, fibreuse ou filamenteuse, peut être du type tissée, tricotée ou non-tissée.

Si certes, les propriétés hémostatiques de contact et cicatrisantes de l'acide polyuronique ou de ses composés sont connues, en revanche sa mise en oeuvre en qualité de gaine de renfort de sutures faisant corps avec celles-ci, n'a jamais été proposée.

Selon l'invention, l'acide polyuronique [C₆H₈O₆⁻]ₙ est mis en oeuvre directement, sous la forme de cellulose oxydée obtenue à partir de végétaux (plantes, bois), ou sous la forme d'alginate de calcium, c'est-à-dire de sels calciques d'acide polyuronique [C₆H₈O₆⁻]ₙ Ca²⁺ₘ.

Avantageusement, cet alginate de calcium est obtenu à partir d'algues.

Textilisation de l'alginate de calcium : on réalise une nappe fibreuse, non tissée, de la manière suivante. On charge une chargeuse-peseuse en fibres d'alginate de calcium de longueur déterminée, ladite chargeuse alimentant une carde. On obtient en sortie de carde un voile, que l'on replie plusieurs fois sur lui-même, afin de former un tapis, dont on assure la cohésion par aiguilletage, entrelaçage ou encore par jet de fluide. On obtient de la sorte un feutre non tissé, de densité relativement importante, propre à conférer à l'implant une résistance mécanique élevée en vue de remplir sa fonction de renfort de sutures. Néanmoins, cette densité n'est pas trop élevée de telle sorte à conférer à l'implant une élasticité, et une malléabilité telles que l'on aboutit à une parfaite adaptation au support tissulaire et, en corollaire, à l'étanchéité sanguine et gazeuse optimum recherchée.

La textilisation de la cellulose fait appel au tricot, qui offre des propriétés mécaniques voisines. Elle subit ensuite un traitement chimique d'oxydation.

Ce feutre ou ce tricot peuvent être utilisés en attelles planes, découpées à la demande aux dimensions souhaitées. En outre, ils sont d'une souplesse telle qu'il peuvent se présenter sous la forme de gaines ou de manchons, propres à venir se mettre en place par simple coulissement au niveau des deux branches d'une pince pour suture d'un type en soi connu, et tel que commercialisés par exemple sous la marque déposée SEAMGUARD par W.L.Gore & Associates, Inc., ou encore sous la marque déposée PERIPATCH par Mitroflow International, Inc..

Avantageusement, les gaines de renfort ainsi obtenues sont étanchéifiés par imprégnation, et notamment par trempage dans une solution aqueuse d'un ester d'acide polyuronique ou de ses sels, et par exemple d'une solution d'ester d'alginate de sodium à 3%. Cette solution aqueuse d'ester d'alginate de sodium est réalisée à base de Propylène Glycol Alginate, disponible dans la pharmacopée.

Après cette étape d'imprégnation, lesdits implants sont séchés en atmosphère stérile.

De la sorte, on obtient des gaines de renfort de sutures annihilant quasi-complètement les fuites aériques et liquidiennes au niveau desdites sutures.

De tels gaines de renfort de sutures sont tout particulièrement indiqués pour les sutures des tissus, notamment pulmonaires dans le cadre d'exérèses pulmonaires lobaires, dans le cadre de la chirurgie de réduction de volume pulmonaire, de la chirurgie de l'emphysème pulmonaire, du pneumothorax, des résections segmentaires et atypiques (WEDGES), des résections de bulles pulmonaires. Dans tous ces cas, l'étanchéité gazeuse et sanguine est requise.

On connaît en effet les problèmes engendrés par ce défaut d'étanchéité, notamment par la nécessité de maintenir le drainage, prolongeant comme déjà dit le séjour du malade à l'hôpital, et grevant de fait de manière significative le coût en résultant, mais également la morbidité.

Cependant, de telles gaines de renfort sont également tout particulièrement adaptées dans le cadre des renforcements de sutures bronchiques, digestives, cardio-vasculaires ou encore uro-génitales, dans lesquelles seule une étanchéité sanguine est requise (les sutures bronchiques requérant également une étanchéité gazeuse).

Dans les différents cadres de mise en place de l'implant conforme à l'invention, celui-ci peut être utilisé :
- soit comme gaine de renfort de suture mécanique à l'aide d'une pince à suture du type de celle mentionnée précédemment, et alors se présenter sous la forme d'une gaine cylindrique ou parallélépipèdique venant s'emmancher sur les deux branches de la pince,
- soit plus traditionnellement comme gaine de renfort de suture manuelle réalisée à l'aide de fils et d'aiguilles, et dans ce cas, il se présente sous la forme de bandes ou bandelettes de forme sensiblement rectangulaire, découpées à la demande au sein d'une nappe de plus grandes dimensions.

La mise en oeuvre d'un tel matériau à base d'acide polyuronique ou de ses sels favorise l'aérostase, l'hémostase et la cicatrisation, aboutissant à une guérison plus rapide des malades.

Sa structure tissée, tricotée ou non tissée permet en outre une conformation à la fabrication adaptée à chacun des modèles d'agrafeuses mécaniques disponibles sur le marché, y compris pour celles utilisées en vidéo-chirurgie ou coeliochirurgie. Selon le type de pince et le type d'agrafage, un ou deux manchons sont nécessaires (enclume et chargeur d'agrafes ou enclume ou chargeur isolés).

Par ailleurs, le matériau mis en oeuvre est tout à la fois biocompatible et résorbable en une durée typique de trois mois.

En outre, compte tenu de l'origine végétale du matériau mis en oeuvre, on obtient une totale innocuité de l'implant, tant vis à vis des virus que vis à vis des prions, lui conférant de fait une sécurité d'utilisation élevée.

Enfin, de part la nature des composés mis en oeuvre, le coût pour la réalisation de tels implants s'avère tout à fait modéré, susceptible ainsi de favoriser leur développement et l'extension des indications d'implantation.

## Revendications

1. Gaine de renfort pour sutures destinée à être mise en oeuvre pour renforcer mécaniquement les zones tissulaires à suturer, **caractérisée en ce qu'**elle est formée d'un manchon en matière textile fibreuse ou filamenteuse obtenue à partir d'acide polyuronique ou de ses sels favorisant la cicatrisation, et **en ce qu'**elle est imprégnée d'un matériau d'étanchéification constitué par un ester d'acide polyuronique en solution aqueuse, ladite gaine présentant une étanchêité par rapport à l'air et aux liquides biologiques et permettant l'adaptation au support tissulaire et l'étanchéité gazeuse et/ou sanguine des sutures.

2. Gaine de renfort pour sutures tissulaires selon la revendication 1, **caractérisée en ce que** la nappe textile est tissée.

3. Gaine de renfort selon la revendication 1, **caractérisée en ce que** la nappe textile est tricotée.

4. Gaine de renfort selon l'une quelconque des revendications précédentes, **caractérisée en ce que** l'acide polyuronique est mis en oeuvre directement, sous la forme de cellulose oxydée obtenue à partir de végétaux.

5. Gaine de renfort selon l'une quelconque des revendications 1 à 3, **caractérisée en ce que** l'acide polyuronique est mis en oeuvre sous forme d'alginate de calcium.

6. Gaine de renfort selon l'une quelconque des revendications précédentes, **caractérisée en ce que** l'alginate de calcium est obtenu à partir d'algues.

7. Gaine de renfort selon l'une quelconque des revendications précédentes, **caractérisée en ce que** l'ester d'acide polyuronique est un ester d'alginate de sodium.

8. Gaine de renfort selon l'une quelconque des revendications précédentes, **caractérisée en ce que** le matériau d'imprégnation a été séché.

9. Gaine de renfort pour sutures tissulaires selon l'une quelconque des revendications précédentes, **caractérisée en ce qu'**elle est parallélépipsdique, destinée à être emmanchée sur les deux branches d'une pince de suture ou sur une seule desdites branches en fonction du type d'agrafage souhaité.

## Patentansprüche

1. Verstärkungsimplantat für Nähte oder Nähfaden zum Einsatz zur mechanischen Verstärkung der zu nähenden Gewebebereiche,
**dadurch gekennzeichnet, dass**
es durch eine Ummantelung aus einem fiberartigen oder faserigen Textilmaterial gebildet ist, das aus Polyuronsäure oder ihren Salzen gewonnen ist, die die Narbenbildung begünstigen, und dass es durch ein Dichtungsmaterial imprägniert ist, das aus einem Polyuronsäureester in einer wässerigen Lösung besteht, wobei das Implantat eine Dichtung gegenüber Luft und biologischen Flüssigkeiten darstellt und die Adaption an einen Gewebeträger und die Undurchlässigkeit für Gase und/oder Blut der Nähte ermöglicht.

2. Verstärkungsimplantat für Gewebenähte nach Anspruch 1,
**dadurch gekennzeichnet, dass**
der Textilmantel gewebt ist.

3. Verstärkungsimplantat für Gewebenähte nach Anspruch 1,
**dadurch gekennzeichnet, dass**
der Textilmantel gewirkt oder gestrickt ist.

4. Verstärkungsimplantat nach einem der vorangehenden Ansprüche,
**dadurch gekennzeichnet, dass**
die Polyuronsäure direkt in der Form einer sauerstoffhaltigen oder oxydierten Cellulose angewendet wird, die aus Pflanzen gewonnen ist.

5. Verstärkungsimplantat nach einem der Ansprüche 1-3,
**dadurch gekennzeichnet, dass**
die Polyuronsäure in Form von Calcium-Alginat angewendet ist.

6. Verstärkungsimplantat nach einem der vorangehenden Ansprüche,
**dadurch gekennzeichnet, dass**
das Calcium-Alginat aus Algen gewonnen ist.

7. Verstärkungsimplantat nach einem der vorangehenden Ansprüche,
**dadurch gekennzeichnet, dass**
das Polyuronsäureester ein Alginatester von Natrium ist.

8. Verstärkungsimplantat nach einem der vorangehenden Ansprüche,
**dadurch gekennzeichnet, dass**
das Imprägniermaterial getrocknet worden ist.

9. Verstärkungsimplantat für Gewebenähte nach einem der vorangehenden Ansprüche,
**dadurch gekennzeichnet, dass**
es parallelepipedisch und dafür bestimmt ist, in Abhängigkeit von dem gewünschten Typ der Klammerung auf den beiden Schenkeln einer Nahtklammer oder auf einem einzigen dieser Schenkel aufgebracht zu werden.

## Claims

1. Reinforcement sheath for sutures intended to be used for mechanically reinforcing tissue zones to be sutured, **characterized in that** it is formed from a sleeve made of fibrous or filamentous textile material obtained from polyuronic acid, or from the salts thereof, promoting cicatrization, and **in that** it is impregnated with a material for making tight consisting of an ester of polyuronic acid in aqueous solution, said sheath exhibiting tightness with respect to air and to biological fluids, and making it possible to adjust to the tissue support and to obtain tightness of the sutures with respect to.

2. Reinforcement sheath for tissue sutures according to Claim 1, **characterized in that** the textile sheet is woven.

3. Reinforcement sheath according to Claim 1, **characterized in that** the textile sheet is knitted.

4. Reinforcement sheath according to any one of the preceding claims, **characterized in that** the polyuronic acid is employed directly, in the form of oxidized cellulose obtained from plants.

5. Reinforcement sheath according to any one of Claims 1 to 3, **characterized in that** the polyuronic acid is employed in the form of calcium alginate.

6. Reinforcement sheath according to any one of the preceding claims, **characterized in that** the calcium alginate is obtained from algae.

7. Reinforcement sheath according to any one of the preceding claims, **characterized in that** the ester of polyuronic acid is a sodium alginate ester.

8. Reinforcement sheath according to any one of the preceding claims, **characterized in that** the impregnation material has been dried.

9. Reinforcement sheath for tissue sutures according to any one of the preceding claims, **characterized in that** it is parallelepipedal, intended to be fitted over the two branches of a pair of suturing forceps or over just one of said branches depending on the type of stapling desired.
